(19) Europäisches Patentamt
European Patent Office
Office européen des brevets

(11) Veröffentlichungsnummer: **0 011 843**
**B1**

(12) # EUROPÄISCHE PATENTSCHRIFT

(45) Veröffentlichungstag der Patentschrift: **15.09.82**

(51) Int. Cl.³: **C 07 C 93/14,** A 61 K 7/13

(21) Anmeldenummer: **79104693.1**

(22) Anmeldetag: **26.11.79**

(54) **Neue Entwicklerkomponenten für Oxidationshaarfarben, deren Herstellung sowie diese enthaltende Haarfärbemittel.**

(30) Priorität: **02.12.78 DE 2852272**

(43) Veröffentlichungstag der Anmeldung: **11.06.80 Patentblatt 80/12**

(45) Bekanntmachung des Hinweises auf die Patenterteilung: **15.09.82 Patentblatt 82/37**

(84) Benannte Vertragsstaaten: **BE CH DE FR GB IT NL**

(56) Entgegenhaltungen:
**DE-A-1 719 381**
**DE-A-2 658 329**
**DE-A-2 741 762**

**Die Akte enthält technische Angaben, die nach dem Eingang der Anmeldung eingereicht wurden und die nicht in dieser Patentschrift enthalten sind.**

(73) Patentinhaber: **Henkel Kommanditgesellschaft auf Aktien, Postfach 1100 Henkelstrasse 67, D-4000 Düsseldorf 1 (DE)**

(72) Erfinder: **Rose, David, Dr., Holbeinweg 7, D-4010 Hilden (DE)**
Erfinder: **Busch, Peter, Dr., Gottfried-August-Bürger-Strasse 10, D-4006 Erkrath-Unterbach (DE)**
Erfinder: **Lieske, Edgar, Hunsrückenstrasse 40, D-4000 Düsseldorf (DE)**

**EP 0 011 843 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

BUNDESDRUCKEREI BERLIN

# Neue Entwicklerkomponenten für Oxidationshaarfarben, deren Herstellung sowie diese enthaltende Haarfärbemittel

Gegenstand der Erfindung sind neue Bis-(2,5-Diaminophenoxy)-alkane der allgemeinen Formel

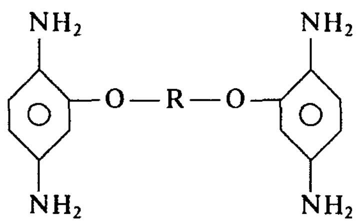

in der R einen geradkettigen oder verzweigtkettigen Alkylenrest mit 1—12 Kohlenstoffatomen darstellt.

Die Herstellung der erfindungsgemäßen Bis-(2,5-Diaminophenoxy)-alkane erfolgt analog zu literaturbekannten Herstellungsverfahren durch Hydrierung entsprechender Bis-(5-Nitro-2-aminophenoxy)-alkane in Gegenwart eines Hydrierkatalysators.

Weitere Gegenstände der Erfindung sind die Verwendung der neuen Bis-(2,5-Diaminophenoxy)-alkane als solcher oder in Gestalt ihrer Salze mit anorganischen oder organischen Säuren als Entwicklerkomponenten in Oxidationshaarfarben sowie Haarfärbemittel, die die neuen Bis-(2,5-Diaminophenoxy)-alkane bzw. deren Salze enthalten.

Für das Färben von Haaren spielen die sogenannten Oxidationsfarben, die durch oxidative Kupplung einer Entwicklerkomponente mit einer Kupplerkomponente entstehen, wegen ihrer intensiven Farben und sehr guten Echtheitseigenschaften eine bevorzugte Rolle. Als Entwicklersubstanzen werden üblicherweise Stickstoffbasen wie p-Phenylendiaminderivate, Diaminopyridine, 4-Aminopyrazolonderivate, heterocyclische Hydrazone eingesetzt. Als sogenannte Kupplerkomponenten werden m-Phenylendiaminderivate, Phenole, Naphthole, Resorcinderivate und Pyrazolone genannt.

Gute Oxidationshaarfarbstoffkomponenten müssen in erster Linie folgende Voraussetzungen erfüllen:

Sie müssen bei der oxidativen Kupplung mit den jeweiligen Entwickler- bzw. Kupplerkomponenten die gewünschten Farbnuancen in ausreichender Intensität ausbilden. Sie müssen ferner ein ausreichendes bis sehr gutes Aufziehvermögen auf menschlichem Haar besitzen, und sie sollen darüber hinaus in toxikologischer und dermatologischer Hinsicht unbedenklich sein.

Die üblicherweise als Entwicklersubstanzen verwendete Verbindungsklasse der substituierten bzw. unsubstituierten p-Phenylendiamine besitzt den Nachteil, daß sie bei einer Reihe von Personen Sensibilisierungen und in deren Gefolge schwere Allergien hervorruft. Die zur Vermeidung dieser dermatologischen Nachteile in neuerer Zeit vorgeschlagenen Entwicklersubstanzen können in ihren anwendungstechnischen Eigenschaften nicht immer voll befriedigen.

Es bestand daher bei der Suche nach brauchbaren Oxidationshaarfarbstoffen die Aufgabe, geeignete Komponenten aufzufinden, die vorgenannte Voraussetzungen in optimaler Weise erfüllen.

Es wurde nun gefunden, daß man zu Oxidationshaarfarben, die den gestellten Anforderungen in besonders hohem Maße gerecht werden, gelangt, wenn man als Entwicklerkomponenten Bis-(2,5-Diaminophenoxy)-alkane der allgemeinen Formel

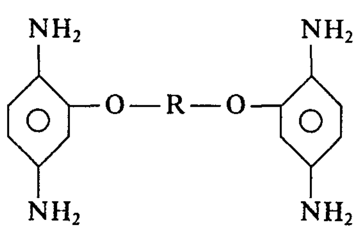

in der R einen geradkettigen oder verzweigtkettigen Alkylenrest mit 1—12 Kohlenstoffatomen darstellt, sowie deren anorganische oder organische Salze in Kombination mit üblichen Kupplersubstanzen verwendet.

Haarfärbemittel auf Basis von Oxidationsfarbstoffen mit einem Gehalt an Bis-(2,5-Diaminophenoxy)-alkanen der allgemeinen Formel

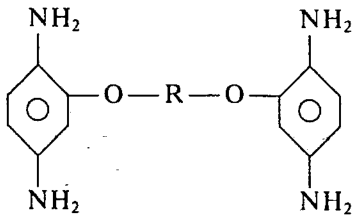

in der R einen geradkettigen oder verzweigtkettigen Alkylenrest mit 1—12 Kohlenstoffatomen bedeutet, sowie deren anorganischen oder organischen Salzen als Entwicklerkomponenten und den in Oxidationshaarfarben üblichen Kupplersubstanzen stellen demnach besonders wertvolle Kompositionen auf dem Gebiet der Oxidationshaarfarben dar.

Besondere Bedeutung ist dabei den Entwicklerkomponenten beizumessen, bei denen gemäß vorgenannter Formel R einen geradkettigen Alkylenrest mit 1—4 Kohlenstoffatomen darstellt.

Bei ihrem Einsatz als Entwicklerkomponenten liefern die erfindungsgemäßen Verbindungen mit den im allgemeinen für die Oxidationshaarfärbung verwendeten Kupplersubstanzen die unterschiedlichsten, sehr intensiven Farbnuancen, wie sie mit diesen Kupplern und den bisher bekannten Entwicklern nicht erzielbar waren und stellen somit eine wesentliche Bereicherung der oxidativen Haarfärbemöglichkeiten dar. Darüber hinaus zeichnen sich die erfindungsgemäßen Bis-(2,5-Diaminophenoxy)-alkane durch sehr gute Echtheitseigenschaften der damit erzielten Färbungen, durch eine gute Löslichkeit im Wasser, eine gute Lagerstabilität und toxikologische sowie dermatologische Unbedenklichkeit aus.

Die erfindungsgemäß als Entwicklerkomponenten zu verwendenden Bis-(2,5-Diaminophenoxy)-alkane können entweder als solche oder in Form ihrer Salze mit anorganischen oder organischen Säuren, wie z. B. als Chloride, Sulfate, Phosphate, Acetate, Propionate, Lactate, Citrate eingesetzt werden.

Als erfindungsgemäß einzusetzende Entwicklerkomponenten sind z. B.

Bis-(2,5-Diaminophenoxy)-methan,
1,2-Bis-(2,5-Diaminophenoxy)-ethan,
1,3-Bis-(2,5-Diaminophenoxy)-propan,
1,4-Bis-(2,5-Diaminophenoxy)-butan,
1,5-Bis-(2,5-Diaminophenoxy)-pentan,
1,6-Bis-(2,5-Diaminophenoxy)-hexan,
1,8-Bis-(2,5-Diaminophenoxy)-octan,
1,10-Bis-(2,5-Diaminophenoxy)-decan,
1,12-Bis-(2,5-Diaminophenoxy)-dodecan,
1,2-Bis-(2,5-Diaminophenoxy)-propan,
1,3-Bis-(2,5-Diaminophenoxy)-2-methylpropan,
1,6-Bis-(2,5-Diaminophenoxy)-2-methylhexan

zu nennen.

Als Beispiele für in den erfindungsgemäßen Haarfärbemitteln einzusetzende Kupplerkomponenten sind α-Naphthol, o-Kresol, m-Kresol, 2,6-Dimethylphenol, 2,5-Dimethylphenol, 3,4-Dimethylphenol, 3,5-Dimethylphenol, Brenzcatechin, Pyrogallol, 1,5- bzw. 1,7-Dihydroxy-naphthalin, 5-Amino-2-methylphenol, Hydrochinon, 2,4-Diamino-anisol, m-Toluylendiamin, 4-Aminophenol, Resorcin, Resorcinmonomethylether, m-Phenylendiamin, 1-Phenyl-3-methyl-pyrazolon-5, 1-Phenyl-3-aminopyrazolon-5, 1-Phenyl-3,5-diketo-pyrazolidin, 1-Methyl-7-dimethyl-amino-4-hydroxy-chinolon-2, 1-Amino-3-acetacetylamino-4-nitro-benzol oder 1-Amino-3-cyanacetylamino-4-nitro-benzol anzuführen.

In den erfindungsgemäßen Haarfärbemitteln werden die Entwicklerkomponenten im allgemeinen in etwa molaren Mengen, bezogen auf die verwendeten Kupplersubstanzen, eingesetzt. Wenn sich auch der molare Einsatz als zweckmäßig erweist, so ist es jedoch nicht nachteilig, wenn die Entwicklerkomponente in einem gewissen Überschuß oder Unterschuß zum Einsatz gelangt.

Es ist ferner nicht erforderlich, daß die Entwicklerkomponente und die Kupplersubstanz einheitliche Produkte darstellen, vielmehr können sowohl die Entwicklerkomponente Gemische der erfindungsgemäß zu verwendenden Bis-(2,5-Diaminophenoxy)-alkane als auch die Kupplersubstanz Gemische der vorstehend genannten Kupplerkomponenten darstellen.

Darüber hinaus können die erfindungsgemäßen Haarfärbemittel andere bekannte und übliche Entwicklerkomponenten sowie auch gegebenenfalls übliche direktziehende Farbstoffe im Gemisch enthalten, falls dies zur Erzielung gewisser Farbnuancen erforderlich ist.

Die oxidative Kupplung, d. h. die Entwicklung der Färbung, kann grundsätzlich wie bei anderen Oxidationshaarfarbstoffen auch, durch Luftsauerstoff erfolgen. Zweckmäßigerweise werden jedoch chemische Oxidationsmittel eingesetzt. Als solche kommen insbesondere Wasserstoffperoxid oder dessen Anlagerungsprodukte an Harnstoff, Melamin und Natriumborat sowie Gemische aus

derartigen Wasserstoffperoxidanlagerungsverbindungen mit Kaliumperoxiddisulfat in Betracht.

Als Entwicklerkomponente besitzen dabei die erfindungsgemäßen Bis-(2,5-Diaminophenoxy)-alkane den Vorteil, daß sie bereits bei oxidativer Kupplung durch Luftsauerstoff befriedigende Färbeergebnisse liefern und somit eine Haarschädigung durch das sonst für die oxidative Kupplung eingesetzte Oxidationsmittel vermieden werden kann. Wird jedoch gleichzeitig neben der Färbung ein Aufhelleffekt am Haar gewünscht, so ist die Mitverwendung von Oxidationsmitteln erforderlich.

Die erfindungsgemäßen Haarfärbemittel werden für den Einsatz in entsprechende kosmetische Zubereitungen wie Cremes, Emulsionen, Gele oder auch einfache Lösungen eingearbeitet und unmittelbar vor der Anwendung auf dem Haar mit einem der genannten Oxidationsmittel versetzt. Die Konzentration derartiger färberischer Zubereitungen an Kuppler-Entwickler-Kombination beträgt 0,2 bis 5 Gewichtsprozent, vorzugsweise 1 bis 3 Gewichtsprozent. Zur Herstellung von Cremes, Emulsionen oder Gelen werden die Farbstoffkomponenten mit den für derartige Präparationen üblichen weiteren Bestandteilen gemischt. Als solche zusätzlichen Bestandteile sind zum Beispiel Netz- oder Emulgiermittel vom anionischen oder nichtionogenen Typ wie Alkylbenzolsulfonate, Fettalkoholsulfate, Alkylsulfonate, Fettsäurealkanolamide, Anlagerungsprodukte von Ethylenoxid an Fettalkohole, Verdickungsmittel wie Methylcellulose, Stärke, höhere Fettalkohole, Paraffinöl, Fettsäuren, ferner Parfümöle und Haarpflegemittel wie Pantothensäure und Cholesterin zu nennen. Die genannten Zusatzstoffe werden dabei in den für diese Zwecke üblichen Mengen eingesetzt, wie zum Beispiel Netz- und Emulgiermittel in Konzentrationen von 0,5 bis 30 Gewichtsprozent und Verdickungsmittel in Konzentrationen von 0,1 bis 25 Gewichtsprozent, jeweils bezogen auf die gesamte Zubereitung.

Die Anwendung der erfindungsgemäßen Haarfärbemittel kann, unabhängig davon, ob es sich um eine Lösung, eine Emulsion, eine Creme oder ein Gel handelt, im schwach sauren, neutralen oder insbesondere alkalischen Milieu bei einem pH-Wert von 8 – 10 erfolgen. Die Anwendungstemperaturen bewegen sich dabei im Bereich von 15 bis 40° C. Nach einer Einwirkungsdauer von ca. 30 Minuten wird das Haarfärbemittel vom zu färbenden Haar durch Spülen entfernt. Hernach wird das Haar mit einem milden Shampoo nachgewaschen und getrocknet.

Die mit den erfindungsgemäßen Haarfärbemitteln erzielbaren Färbungen zeigen unter Einsatz unterschiedlicher Entwickler- und Kupplerkomponenten besonders intensive Farbnuancen. Die erzielten Färbungen haben gute Licht-, Wasch- und Reibechtheitseigenschaften und lassen sich leicht mit Reduktionsmitteln wieder abziehen.

Aus der deutschen Offenlegungsschrift 2 741 762 sind zwar schon 2,5-Diaminophenolether sowie Färbemittel für Keratinfasern bekannt, die solche 2,5-Diaminophenolether enthalten. Diese Verbindungen enthalten jedoch nur eine 2,5-Diaminophenoxy-Gruppe im Molekül und ergeben mit üblichen Kupplersubstanzen Oxidationshaarfarben, die bezüglich ihrer Lichtechtheit nicht voll befriedigen.

Aus der deutschen Offenlegungsschrift 1 719 381 sind auch schon Bis-(2-aminophenoxy)-alkane und Bis-(3-aminophenoxy)-alkane bekannt. Hinweise auf eine Eignung als Entwicklerkomponenten in Oxidationsfarbstoffen oder Haarfärbemitteln sind dieser Druckschrift nicht zu entnehmen.

Die nachfolgenden Beispiele sollen den Erfindungsgegenstand näher erläutern, ohne ihn jedoch hierauf zu beschränken.

## Beispiele

Zunächst wird nachstehend die Herstellung der erfindungsgemäßen Bis-(2,5-Diaminophenoxy)-alkane beschrieben.

Die Herstellung der als Zwischenprodukte dienenden Bis-(5-Nitro-2-aminophenoxy)-alkane erfolgte gemäß den Angaben der deutschen Offenlegungsschrift 2 658 329 durch Umsetzung von 2 Mol des entsprechend substituierten Nitroacetaminophenols mit etwas mehr als 1 Mol Dibromalkan in Gegenwart von etwas weniger als 2 Mol Alkalihydroxid in wäßrig-alkoholischem Medium bei Temperaturen zwischen 100 und 150° C zu den Bis-Nitroacetaminoverbindungen und anschließende Abspaltung der Acetylreste.

### A) Bis-(2,5-Diaminophenoxy)-methan-tetrahydrochlorid

10 g Bis-(5-Nitro-2-aminophenoxy)-methan wurden in 250 ml Ethanol in Gegenwart von 0,5 g Katalysator (5% Palladium auf Kohle) bei 70° C und 100 bar hydriert. Nach beendeter Wasserstoffaufnahme wurde vom Katalysator abfiltriert und mit verdünnter Salzsäure angesäuert. Die Lösung wurde zur Trockne eingeengt und der Rückstand bei 70° C getrocknet. Es wurden 10,8 g an Bis-(2,5-Diaminophenoxy)-methan-tetrahydrochlorid vom Schmelzpunkt > 330° C erhalten.

B) 1,2-Bis-(2,5-Diaminophenoxy)-ethan-tetrahydrochlorid

Entsprechend den Angaben unter A wurden 13 g 1,2-Bis-(5-Nitro-2-aminophenoxy)-ethan katalytisch reduziert und aufgearbeitet. Es wurden 8,9 g an 1,2-Bis-(2,5-Diaminophenoxy)-ethan-tetrahydrochlorid erhalten, das bei ca. 250° C unter Zersetzung schmolz.

C) 1,3-Bis-(2,5-Diaminophenoxy)-propan-tetrahydrochlorid

Entsprechend den Angaben unter A) wurden 10 g 1,3-Bis-(5-Nitro-2-aminophenoxy)-propan bei 80° C und 60 bar katalytisch reduziert und aufgearbeitet. Es wurden 10 g an 1,3-Bis-(2,5-Diaminophenoxy)-propan-tetrahydrochlorid vom Schmelzpunkt 289 – 293° C erhalten.

D) 1,4-Bis-(2,5-Diaminophenoxy)-butan-tetrahydrochlorid-monohydrat

Entsprechend den Angaben unter A) wurden 14 g 1,4-Bis-(5-Nitro-2-aminophenoxy)-butan bei 80° C und 100 bar katalytisch hydriert und aufgearbeitet. Es wurden 13,6 g an 1,4-Bis-(2,5-Diaminophenoxy)-butan-tetrahydrochlorid-monohydrat vom Schmelzpunkt 278 – 280° C erhalten.

Die vorgenannten, in ihrer Herstellung beschriebenen Bis-(2,5-Diaminophenoxy)-alkane wurden als Entwicklerkomponenten in den folgenden Beispielen eingesetzt.

Als Kupplerkomponenten dienten folgende Substanzen:

| | |
|---|---|
| K1: | α-Naphthol |
| K2: | Resorcin |
| K3: | 2,7-Dihydroxynaphthalin |
| K4: | m-Aminophenol |
| K5: | 2,4-Dichlor-3-aminophenol |
| K6: | 2,6-Diaminotoluol |
| K7: | 2-Methylresorcin |
| K8: | 1,7-Dihydroxynaphthalin |
| K9: | 4-Chlorresorcin |
| K10: | 1,5-Dihydroxynaphthalin |
| K11: | 2,4-Diaminophenol |
| K12: | 2,6-Dihydroxy-4-methylpyridin |
| K13: | N,N-Bis-(β-hydroxyethyl)-m-phenylendiamin |
| K14: | 1-Amino-3-di-(β-hydroxyethylamino)-4-ethoxybenzol |

Die erfindungsgemäßen Haarfärbemittel wurden in Form einer Cremeemulsion eingesetzt. Dabei wurden in eine Emulsion aus

10 Gew.-Teilen Fettalkoholen der Kettenlänge $C_{12} - C_{18}$,
10 Gew.-Teilen Fettalkoholsulfat (Natriumsalz, Kettenlänge $C_{12} - C_{18}$) und
75 Gew.-Teilen Wasser

jeweils 0,01 Mol der in der nachstehenden Tabelle aufgeführten Entwicklersubstanzen und Kupplersubstanzen eingearbeitet. Danach wurde der pH-Wert der Emulsion mittels Ammoniak auf 9,5 eingestellt und die Emulsion mit Wasser auf 100 Gewichtsteile aufgefüllt. Die oxidative Kupplung wurde entweder mit Luftsauerstoff oder mit 1%iger Wasserstoffperoxidlösung als Oxidationsmittel durchgeführt, wobei zu 100 Gewichtsteilen der Emulsion 10 Gewichtsteile Wasserstoffperoxidlösung gegeben wurden. Die jeweilige Färbecreme mit oder ohne Zusatz von Oxidationsmitteln wurde auf zu 90% ergrautes, nicht besonders vorbehandeltes Menschenhaar aufgetragen und dort 30 Minuten belassen. Nach Beendigung des Färbeprozesses wurde das Haar mit einem üblichen Haarwaschmittel ausgewaschen und anschließend getrocknet. Die dabei erhaltenen Färbungen sind nachstehender Tabelle 1 zu entnehmen.

Tabelle 1

| Beispiel | Entwickler | Kuppler | Nuance des gefärbten Haares durch Luftoxidation | Nuance des gefärbten Haares 1%ige $H_2O_2$-Lösung |
|---|---|---|---|---|
| 1 | A | K1 | violettgrau | dunkelviolett |
| 2 | A | K2 | braungrau | braungrau |
| 3 | A | K3 | silbergrau | rotgrau |
| 4 | A | K4 | silbergrau | rauchfarbig |
| 5 | A | K5 | violett | dunkelviolett |
| 6 | A | K6 | blau | blau |
| 7 | A | K7 | braun | dunkelbraun |
| 8 | A | K8 | dunkelviolett | dunkelviolett |
| 9 | A | K9 | braun | braun |
| 10 | B | K1 | dunkelblau | dunkelblau |
| 11 | B | K2 | braungrau | rotgrau |
| 12 | B | K3 | braungrau | violettgrau |
| 13 | B | K4 | braun | braun |
| 14 | C | K1 | dunkelblau | dunkelviolett |
| 15 | C | K2 | braungrau | rotgrau |
| 16 | C | K3 | braun | blau |
| 17 | C | K4 | silbergrau | maulwurfgrau |
| 18 | C | K9 | braungrau | braun |
| 19 | C | K7 | purpur | rot |
| 20 | C | K10 | blau | blaugrau |
| 21 | C | K11 | dunkelbraun | braun |
| 22 | C | K12 | blau | blaugrau |
| 23 | C | K5 | dunkelblau | dunkelblau |
| 24 | C | K13 | blau | dunkelblau |
| 25 | C | K14 | dunkelblau | blaugrau |
| 26 | D | K1 | violettgrau | dunkelblau |
| 27 | D | K2 | rot | graurot |
| 28 | D | K3 | braun | violett |
| 29 | D | K4 | braungrau | blau |

**Patentansprüche**

1. Bis-(2,5-Diaminophenoxy)-alkane der allgemeinen Formel

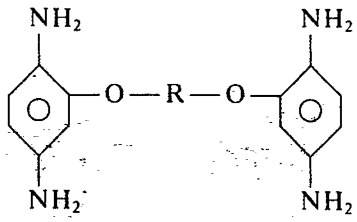

in der R einen geradkettigen oder verzweigtkettigen Alkylenrest mit 1—12 Kohlenstoffatomen darstellt.

2. Bis-(2,5-Diaminophenoxy)-alkane gemäß Anspruch 1, dadurch gekennzeichnet, daß R einen geradkettigen Alkylenrest mit 1—4 Kohlenstoffatomen darstellt.

3. Verfahren zur Herstellung der Bis-(2,5-Diaminophenoxy)-alkane gemäß Anspruch 1 und 2 durch Hydrierung entsprechender Bis-(5-Nitro-2-aminophenoxy)-alkane in Gegenwart eines Hydrierkatalysators.

4. Verwendung von Bis-(2,5-Diaminophenoxy)-alkanen nach Anspruch 1 und 2 sowie deren Salzen mit anorganischen oder organischen Säuren als Entwicklerkomponenten in Oxidationshaarfarbstoffen.

5. Oxidationshaarfärbemittel mit einem Gehalt an Bis-(2,5-Diaminophenoxy)-alkanen gemäß Anspruch 1 und 2 sowie deren Salzen mit anorganischen oder organischen Säuren als Entwicklerkomponenten und den in Oxidationshaarfarben üblichen Kupplersubstanzen.

6. Haarfärbemittel nach Anspruch 5, gekennzeichnet durch einen Gehalt an Entwickler-Kuppler-Kombinationen in einer Menge von 0,2 bis 5 Gewichtsprozent, vorzugsweise 1 bis 3 Gewichtsprozent, bezogen auf das gesamte Haarfärbemittel.

**Claims**

1. Bis-(2,5-diaminophenoxy)-alkanes corresponding to the following general formula

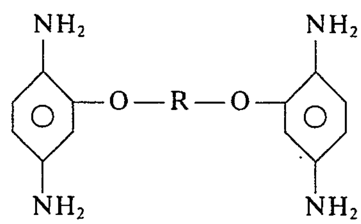

in which R represents a straight-chain or branched-chain alkylene radical containing from 1 to 12 carbon atoms.

2. Bis-(2,5-diaminophenoxy)-alkanes as claimed in Claim 1, characterised in that R represents a straight-chain alkylene radical containing from 1 to 4 carbon atoms.

3. A process for producing the bis-(2,5-diaminophenoxy)-alkanes claimed in Claims 1 and 2 by hydrogenating corresponding bis-(5-nitro-2-aminophenoxy)-alkanes in the presence of a hydrogenation catalyst.

4. The use of the bis-(2,5-diaminophenoxy)-alkanes claimed in Claims 1 and 2 and their salts with inorganic or organic acids as developer components in oxidative hair dyes.

5. Oxidative hair dyes containing the bis-(2,5-diaminophenoxy)-alkanes claimed in Claims 1 and 2 and their salts with inorganic or organic acids as developer components and the coupler substances normally used in oxidative hair dyes.

6. Hair dyes as claimed in Claim 5, characterised by a content of developer/coupler combinations of from 0.2 to 5% by weight and preferably of from 1 to 3% by weight, based on the hair dye as a whole.

**Revendications**

1. Bis-(2,5-diaminophénoxy)-alcanes de formule générale:

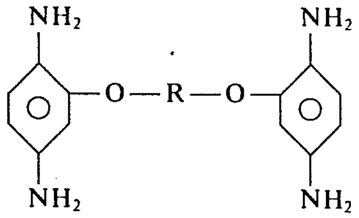

dans laquelle R représente un radical alcoylène à chaîne droite ou ramifiée ayant 1 à 12 atomes de carbone.

2. Bis-(2,5-diaminophénoxy)-alcanes selon la revendication 1, caractérisés en ce que R représente un radical alcoyl ène à chaîne droite ayant 1 à 4 atomes de carbone.

3. Procédé de préparation des bis-(2,5-diaminophénoxy)-alcanes selon les revendications 1 et 2 par hydrogénation des bis-(5-nitro-2-aminophénoxy)-alcanes correspondants en présence d'un catalyseur d'hydrogénation.

4. Utilisation de bis-(2,5-diaminophénoxy)-alcanes selon les revendications 1 et 2 ainsi que de leurs sels avec des acides minéraux ou organiques comme composants de développement dans des colorants d'oxydation pour cheveux.

5. Colorants d'oxydation pour cheveux ayant une teneur en bis-(2,5-diaminophénoxy)-alcanes selon les revendications 1 et 2 ainsi que de leurs sels avec des acides minéraux ou organiques comme composants de développement et avec les substances de couplage en usage dans les teintures de cheveux par oxydation.

6. Agents de teinture des cheveux selon la revendication 5, caractérisés par une teneur en combinaisons agent de développement-coupleur en une quantité de 0,2 à 5% en poids, de préférence de 1 à 3% en poids, par rapport à l'agent de teinture pour cheveux dans son ensemble.